# EUROPEAN PATENT APPLICATION

(11) **EP 2 090 315 A1**
(43) Date of publication of application: **19.08.2009**
(21) Application number: 07011585.2
(22) Date of filing: 13.06.2007
(51) Int. Cl.: A61K 35/00, C12G 3/00, A61K 36/00, A61K 36/06, A61K 45/00

(54) **Method and system for producing medicinal alcohol as a prophylatic or remedy for cancer, HIV, AIDS and autoimmune diseases**

(71) Applicant: Kapur MBBS, B., Dr., South Yorkshire S66 8QF (GB); Kapur, Rajesh, Maltby, Nr Rotherham S. Yorkshire S66 8QF (GB)
(72) Inventor: Kapur MBBS, B., Dr., South Yorkshire S66 8QF (GB); Kapur, Rajesh, Maltby, Nr Rotherham S. Yorkshire S66 8QF (GB)

(57) **Abstract**

A method and system for producing a prophylatic or a remedy for both solid and blood Cancer, HIV, AIDS, and Autolmmune Diseases by means of a malic, latic acid naturally fermented pasteurised solution comprises the extract of mushrooms, fruits , fruit juices, seeds , beans, various aruvedic herbs and flowers which can be taken as a dietary supplement, in both a non-toxic or a toxic variety.

## Description

### Field of Invention

To understand the approach it is first necessary to understand the nature of the problem. Cancer is a disease characterized by the uncontrolled division of cells and the ability of these to spread by either direct growth or metastasis ( i.e. through the blood or lymphatic system to other parts of the body.)

HIV primarily affects vital cells in the human immune system such as helper Tcells, when the body is low on these the disease develops. Low levels of CD4 Tcells develop through three main mechanisms. Firstly, viral killing of infected cells by T cells. Secondly, increased rate of apoptosis in infected cells, and finally the killing of Infected CD4 Tcells by CD8 cytotoxic lymphocytes (killer Tcell), that recognise infected cells.

Autoimmune diseases where bodily function is effectively shutdown by an overactive immune system.

All of the problems above have the immune system in common , or perhaps the inability of the immune system to cope, or the suppression of it or the nervous system through physiological, and psychological changes.

An increase in the resistance is most important, in the development of a therapy therefore we shall also focus on this and the natural regeneration of the body and it is this wherein we believe the answer lies.

Most of the work in this application admittedly based upon observation and the findings of people who have done significant research in related areas and that which is public knowledge. The combination of, and the insight applied is , however, completely unique, because no one has ever before sought to combine the whole, or considered combining the whole in this slant this has not been obvious save perhaps our forefathers and that that has been lost in myth, however we believe the solution is now in sight.

### The references used:

An English Translation of, The Rig Veda (the Earliest of the Holy Books of the Hindus the last tribe to still observe some of the Aryran Traditions) which describes a ceremony in which self sacrifice was carried out. The Aryrans drank Soma were intoxicated and felt at one with the Gods.

Dr Max Gerson a German Doctor, who used a nutritional healing therapy for cancer diseases, especially his use of juicing and enemas, as well as the importance of pancreatic enzymes.
the references used is
http://www.healingdaily.com/conditions/Gerson-therapy.htm.
Other references used:-
Gordon Wasson and his article Soma the Divine Mushroom of Immortallity.
http://www.botany.hawaii.edu/faculty/wong/BOT135/Lect20a.htm.

PCT/KR2001/002090 , WO2002/090559 (Owner BIOHUB CO. LTD REPUBLIC of KOREA.)
A Method for preparing Lactic Acid fermented solution of mushroom and lactic acid fermented solution of mushroom.
The inventors in the above reference's finding were that the mushroom inoculated with lactic acid strain helped the immune system, in combating Cancer in particular.

CA 2378402 METHOD OF TREATING MALIGNANCIES AND VIRAL INFECTIONS AND IMPROVING IMMUNE FUNCTION WITH A DIETARY SUPPLEMENT SUN ALEXANDER S. SUN FARM CORP UNITED STATES,The invention relates to treating malignancies and restoring CD4 cell levels increasing NK Activity and suppressing tumour growth.
The inventors in the above reference's findings relating to an extract of the common mushroom and mung beans and soya bean.

CA 2285900 W098/44937 PREVENTIVE OR REMEDY FOR KIDNEY DISEASES
The inventors in the above reference discovered a prophylatic and remedy for renal Disease, by use of a hydrophilic solvent extract of a mushroom having renal function improving effect.

CA 2461661 USE OF A HISTAMINE TO TREAT LIVER DISEASE MAXIM PHARACEUTICALS , INC (United States).This reference relates to methods for treating and preventing hepatic tissue and cell damage caused by reactive oxygen species in mammals, More specifically the invention relates to the prevention and/or reduction and/or reversal of hepatic tissue and cell damage through the administration of histamine and histamine related compounds.

LOSS OF REGENERATIVE CAPACITY: A TRADE OFF FOR IMMUNE SPECIFICITY ? A. L Mescher & A.W. Neff Center for Regenerative Biology and Medicine, Department of Anatomy and Cell Biology Indiana University School of Medicine.

Further References used are:-

The Wikapedia a web based Encyclopaedia
http://www.chauvre-info.ch/info/en/quinonoid-cannabinoids-are-they.html
Advances in Immunology Vol 70 Academic Press 1998
Interleukin-12 A cytokine at the interface of Inflammation and Immunity
http://tsansenterprise.com/liver.htm
http://newsvote.bbc.co.uk/2/hillhealth/6563043.stm (Triphala)
Pathology JCE Underwood.
http://www.himalayahealthcare.com

### Background of the Invention

This application provides two advantages over the prior art in regards to Mushrooms. The first advantage is its shows a new way of making non-toxic mild alcohol. The invention then uses this method to at the same time to extract nutrients from a variety of vegetables and plants , herbs and flowers giving a mild drink nutritionally benefical for the heightening immune system i.e. it allows the resistance of the subject and the raw material for regeneration to be present in the extract but also the mild psychological effect of the alcohol itself. It perhaps mimicks the way Alchemy was carried out centuries ago. The alcohol drink made then certainly did not resemble the alcohol drinks made today, the alcohol drinks of today are themselves harmful to the liver.

The second advantage of this invention is seen in the toxic version or second embodiment of the alcohol. Chemotherapy is a way of reducing or poisoning cancer cells, the only problem is other cells are affected too currently the body weakens further. The second embodiment describes a process of using poison (a new chemotherapeutic agent) along with its antidote in the same mixture, together with all the beneficial food extracts, in order to cause the conditions for regeneration, by stimulating dendrite cells to be activated and increasing resistance. Finally the creation of a psychological effect to allow the regeneration to continue, by lessening control of the brain on adaptive immune reactions, and stimulating non-adaptive immune functions, to pass on information to the adaptive functions, and to get involved in the process of fighting disease and regeneration.

The final embodiment is based upon the second and is understandable given the nature of the poison and psychotropic nature contained in the byproducts (urine and the faeces) could be used together with the beneficial food extracts as medicine, certainly for patients with bad liver and kidney complaints. Poison is understandably still poison therefore the quicker the body can process the poison the less the impact on the body. Processed poisions already passed through the body of a person would pass through the body of another quicker because they have already been processed.

[One wonders could this have been idea behind the blood and body of Christ ?, whether this was a second sacrifice by a condemned man or by the ancient Aryans in their ceremonies of self sacrifice of handing food and drink to worshippers ? ] or in the urine drinking ceremonies, observed in 1730 by a Swedish Army Officer Filip Johann von Strahlenberg whilst held captive in Siberia by the Russians [Wassons Article]].

To understand the nature of the application it is important to study history, history is important in every walk of life. For example, when a patient walks in with a complaint how can you treat him if you don't know the history of the problem, its important also to understand the essence of the problem and then formulate a solution.

So first going back in time. There was not always labs and biochemists, once upon a time, Alchemy was practised widely. Alchemy is in fact the basis of modern day chemistry, and medicine.

Few know that Asprin is the extract of the bark of a willow tree, the active component has been isolated and is now synthesized. Or that Penicillin comes from the blue mould that can be found on bread.

Botany, alchemy, and observation is the basis of modern day medicine, which has today extended to the lab. When A.Fleming made his observation he had to wait for clinical tests due to the toxic nature of the mould.

Medicines were extracted from plants since ancient times by means of both Water and Alcohol.Our belief was always that it wasn't the same alcohol that we drink today, on reading the article from Wasson it was confirmed, in the inference that the Mushroom drink was replaced by Alcohol introduced by the Russians.

The History in this case, begins 4000 years before Christ, with a people known as the Aryans, and the religious ceremonies practised by them. Focus briefly on the religious holy books and the ceremonies of the Aryans now somewhat diluted but still practised by the descendants of the Indo Ayrans the Hindu's.

The Rig Vedas the first of the holy books of the Hindu's, and describes the aryran ceremonies , the identity of the plant known as Soma, had been lost, until Wasson uncovered it as Amantia Muscaria in 1968. The observations he made are entirely correct, and it's worth reading his article.

Medically, (an observation) poisons already passed through the digestive tract of another would cause less damage to the drinker as they would be already in a soluble enough form to pass through the body quickly useful in treating those with weakened liver and kidney complaints [Reference Wasson's article of Filip Johann van Strahlenberg's observation of urine drinking ceremony]. You can understand now why self sacrifice was required in the old days, especially if more dangerous mushrooms were to be used from the Amantia family.

The Havan ceremony is still today carried out by the Hindu's and is an Aryran religious ceremony, in which fruits, vegetables/food, seeds, herbs and flowers (in olden days and still as in Chinese Medicine tree bark and leaves also) are offered up, some are eaten as a gift from God, water placed alongside is also drunk again as a gift from god.Something else noted whilst observing the elders in my own family, every part of a plant was used for example a peas pod the peas are extracted and the pods are skinned and used in the same food preparation, or another food preparation.

Dr. Max Gerson's work was read with great interest, he was for sure a Doctor whose observations have helped many, a man who had discovered that that nutrients would be more quickly absorbed into the body in fluid form (in the form of juices and especially his work with enemas. When an enema is used it is absorbed into the body and goes directly to the liver where it becomes a very strong detoxicant. It causes the liver to produce more bile, which contains processed toxins and moves bile out towards the small intestine for elimination, this process frees up the liver to process more incoming toxic materials that have accumulated in the organs , tissues and bloodstream. Interesting to hear also that he himself died of poisoning.

Milkthistle available to the Ancient Aryans was not only an antidote to the poisons contained in the Amantia family of mushrooms, but also served as an enema, the leaves of the milkthistle could be used as a spinach substitute. The first embodiment in which a substitute mushroom was added, it was noted that the final solution smelt like spinach , probably at the point after indole -3- carbinol was released, notably an active component in the fight against cancer.

The reason why the second and third embodiments have not yet been tested is because they are toxic and would need to be tested in a lab, in clinical trails on animals and then on humans again to determine the correct dosage depending on the age, metabolism, physiology and the condition of the patient..

After reading the paper on Histamines and Liver Disease (CA 2461661 as above). I became sure that A. Muscaria or its family was not used alone for psychological reasons but a combination of different psychoactive components were used, this would explain the ecstasy.

On reading LOSS OF REGENERATIVE CAPACITY: A TRADE OFF FOR IMMUNE SPECIFICITY as referenced above it confirms observations.

These were that the humans were highly evolved beings and that the immune system was controlled by the brain, and the thinking of the individual himself.

Having therefore examined history and understood the observations of others coupled, with interpretation and observations of the Patent Papers which supported this interpretation and shows of all the work that was carried out on these diseases.

The conclusion became clearer in order to treat these terrible diseases it would be necessary to stimulate the inactive immune system and allow regeneration of course extra nutrients would help in the regeneration, but also a need to stop or change the response of the brain. Before the immune system would respond it would be necessary to stimulate a response of the non-adaptive immune system the dendritic cells whilst inhibiting reaction to other nerve mechanisms that stop regeneration.

It is appreciated that this invention could additionally be used for the purpose of manufacture of a non-toxic medicinal alcoholic drink, as described in the first embodiment, to improve general health of humankind. An example of the invention will now be described in detail with reference to the accompanying drawings in which;

### Drawings

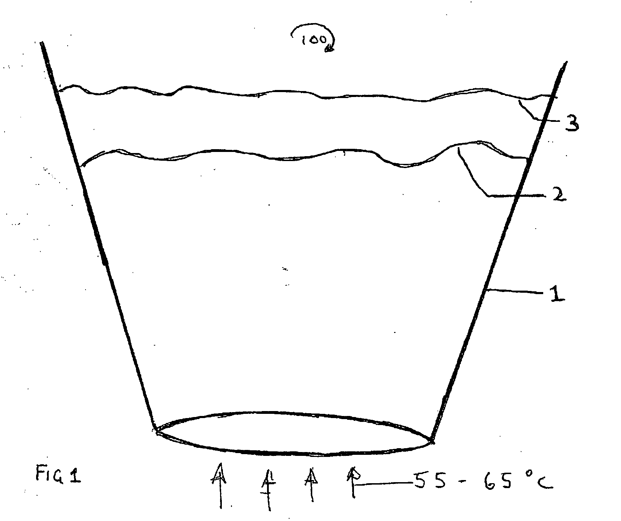

Figure 1 is a schematic diagram of a pot in which various mushrooms, plants, flowers, seeds, kernels, tree bark are pasteurized in a solution of organic acids

### Description of the Invention

This invention has only one primary purpose to relieve the suffering of the many and to provide a new therapy to fight live endangering diseases, such as Cancer, HIV/AIDs and to alleviate autoimmune class of diseases such as Crohn's disease.The immune system is the bodies way of protecting itself against foreign substances. The fundamental protective actions involve, neutrophils, macrophages, killer cells, and T and B cells. An immune system dysfunction can be deemed to be overactive or under active. An under active immune system shows itself in conditions such as Cancer and Aids.

This work centres on the immune cells known as macrophages(also called a monocyte) when this comes into contact with a cancerous or pre-cancerous cell. The macrophage begins to digest the tumour cell. There are two actions which occur at this point either the macrophage can transmit little pieces of the tumour to another cell or it can transform itself into a dendritic cell, there is more and more evidence that the second is the more likely solution. The dendritic cell when fully mature transmits information about the tumour contained in the small digested pieces to the rest of the system through the lymph nodes. I believe that in a cancerous patient that the Dendritic cells have not fully matured hence cannot hand off information to T Lymphocytes or CTLs.

In other words the adaptive immune system is composed of highly specialised, systemic cells and processes that eliminate pathogenic challenges, This specific immune system is activated by the non-specific and older innate immune system namely the Dendrite cells (which is the major system of host defence against pathogens in nearly all other living things).

Hiv , attacks the adaptive CD4+ Tcells precisely the cells that could drive the destruction of the virus but also the cells that drive immunity against all other pathogens encountered during an organisms lifetime.

The purpose of this invention is therefore to combat the lack of CD4+ Tcells , immature Dendritic cells, by using inducing Inflammation in vivo by introduction of a dangerous toxic poison, whilst protecting the body as much as possible against the poison and its effect to force the bodies innate immunity to the fore.

In Humans this means inducing Phagocytosis the control of inflammation. Phagocytosis is performed by specialised cells called phagocytes, which are able to remove foreign bodies and thus fight infection, In humans and many other animals phagocytes include macrophages , monocytes, dendritic cells and granulocytes, this process increases the amounts of interleukin 12 generated, which can help naive Tcells towards th1 type cells using cell to cell communication which take place at a distance via cytokines.

In basic terms to force a response of the immune system, to enhance its ability to fight disease. The immune system does not fight the poison itself, only the inflammation which can also be dealt by neighbouring cells in a tissue which will phagocytize there apoptotic neighbours thus maintaining tissue homeostasis. Thereby leaving the newly activated cells to fight the diseases within the body.
Bacterial preparations have been used in the past, and furthermore there has been sporadic observations since the 1700's of certain Patients undergoing bacterial infections and a commitant remission of the malignant growth.

More activated Dendritic cells stimulated by Psychological responses of substances taken allowing them to mature and activate more of the adaptive immune system. The Psychological response has also a secondary function to inhibit the brain from controlling the adaptive system, allowing regeneration to occur.

For the activation of the adaptive immune response, phagocytosis is a necessary activation step, a T helper Cell must be presented foreign particles bound to the major histocomptability complex class II (MHC II) Receptor to become activated.

Note that MHC I and MHC II treatment therapy has been used in the fight against HIV/AIDS and autoimmune diseases. The psychological response functions to inhibit the overactive immune system.

The advantages of this application are many today the immune system of the body and the invocation of bodies own natural defences are being looked at more and more to provide the answer.

We believe that this method once properly clinically tested will revolutionise Medical Science, as the discovery of Penicillin. All the research to support it is in the references above but to string everything together to produce the solution is not obvious, if it was it would have been brought to the fore in fact this was a journey of discovery.

Accordingly, in a first embodiment of the invention there is provided a method and system for producing a oral supplement, a non toxic mushroom drink to the body, whilst providing all the nutrients and immunomodulators, the body requires for regeneration and apoptosis, and to give a boost to the immune system whilst inducing a physcological response for pleasurable purposes.

Accordingly, in a second embodiment of the invention there is provided a method and system for producing a oral supplement, introducing, and protecting against and quickly removing a poison from the body, whilst providing all the nutrients and immunomodulators, the body requires for regeneration and apoptosis, this to issue both a wake up call and to stimulate both the innate and adaptive immune system whilst inducing a physcological response to change the control signals sent to the immune system.

Accordingly, in the preferred and third embodiment of the invention there is provided a method and system for producing a oral supplement, introducing, and protecting against and quickly removing a poison by product (of the urine and or faeces) of another from the body, whilst providing all the nutrients and immunomodulators, the body requires for regeneration and apoptosis, this to issue both a wake up call and to stimulate both the innate and adaptive immune system whilst inducing a physcological response to change the control signals sent to the immune system.

Figure 1 shows a pot incorporating pasteurised extract and food 100 according to embodiments of the invention.

In the first embodiment of the invention a non-toxic slightly alcoholic pasteurised mushroom drink is produced in a pot 1 for boosting the general health and the immune system in which a small amount of ghee (purified butter) or/ and coconut oil is added to activate the breakdown of the other ingredients 2 which include ingredients contained in the three groups described below immersed fully in the fruit juices /organic acids group as described below, and pasteurised by heating.

The first group being mushrooms wherein the mushroom is selected from the group consisting ofGanderma lucidum, Grifola frondosa, Pleurutus asteraius , Rifivingia applanata, , Agaricus bisporous, Agaricus blazei,lentinus edodes, but the mushroom preferably used is the Gaderma lucidum or the 'Reshi Mushroom' although a combination of mushrooms may be used from the genus's stated.

The second group consists of one or more of the following but preferably a combination of the whole milk thistle plant (bioflavanoid), spinach leafs, mustard leaf spinach, methi leaves , broccoli (3,3 Diindolylmethane,sulforaphane) , wheatgrass, the potato, the tomato, the whole dandelion flower, turnip (shelgum) asparagus, carrot, leek, apricots and their kernels, apples, bananas, pears, flaxseed flower, goji berry, a little coffee.

The third group consists of one or more of the following but preberably a combination of all the ayurvedic plants , herbs , spices, fruits and tree barks, and seeds/ kernels listed below but not limited to those listed below :
1. Rauwolfia serpentina (Sarpagangha) contains Reserpine ( for central nervous system).
2. Castor oil
3. Rosemarinus officinalis Rosemary
4. Santalum album (Chandana or Sandalwood tree contains Santalol anti inflammatory.
5. Saraca asoca (Ashoka Gandhapushpa or Ashoka Tree) contains the estrogenic compound ketosterol.
6. Saussurea lappa (Kushtha or Costus) Anti inflammatory.
7. Saxifraga ligulata (Pasanabheda)
8. Sesaum indicum (Tila, or Sesame Gingelly) anti inflammatory.
9. Sida cordifolia (Bala, Vatya or Country Mallow) contains Ephedrine
10. Slanum nigrum (Kakamachi or Black Nightshade ) this plant is effective in the treatment of cirrhosis of the liver and has shown hepatoprotective activity.
11. Solanum xanthocarpum (Kantakari or Yellow Berried Nightshade)
12. Symlocos racemosa (Lodhra or Lodh Tree) The astringent bark is useful in the treatment of diarrhea, dysentery and liver complaints the stem bark is has anti-inflammatory properties
13. Syzygium aromaticum the clove
14. Syzygium cumini Black Plum or Jambu
15. Vanda roxburghii (Rasna) Anti Inflammatory.
16. Withnia Somnifera (Ashvagandha or winter cherry )
17. Foeniculum vulgare (Shatapushpa or Fennel)
18. Tecomella undllata (rohi, rohitaka or the Rohida tree) the bark contains tecomin it is used for liver disorders.
19. Terminalia arjuna (Arjuna) an agent in hypertension and ischemic heart disease ,it also has a tonic effect in cases of cirrhosis of the liver.
20. Terminalia bellirica (Vbhitaki or Kalidruma or Belliric Myrobalan) part of Triphala.
21. Terminalia Chebula ( Haritaki or Chebula Myrobalan) (part of Triphala) known as an adaptogen and hepatoprotective drug it is also an fective purgative and helps remove toxins from the body.
22. Tinospora codifolia (Guduchi) antispasmodic, anti-inflammatory.
23. Tribulus terrestris (Goskshura Small Caltrops)
24. Trigonella foenum-graecum (Methi Fenugreek)
25. Acacia catecatu (Khadira or Cutch Tree ) antibacterial, anti inflammatory and anti-fungal
26. Acacia Nilcota ( Babbula, Babool Indian Gum- Arabic Tree)
27. Achilla millefolium (Biranj asipha or Yarrow) Anti various hepatic disorders.
28. Acorus calamus (Vacha or Sweet Flag) useful in treating glandular and abdominal tumours , liver and kidney complaints
29. Adhatoda zeylanica (Shwetavasa Vasa Vasaka or Malabar Nut)
30. Aegle marmelos (Bilva bael tree)
31. Allium sativum (Lasuna or Garlic)
32. Aloe barbadensis (ghrita-kumari Indan Aloe) antibacterial and anti-fungal and liver ailments
33. Alpina galanga (Rasna, Mahabharivacha) anti-inflammatory.
34. Amomum subulatum (Aindri, Larger or Greater Cardamom) anti-inflammatory.
35. Andrographis paniculata (Bhunima, Yavatika or the creat) used as a blood purifier.
36. Apium graveolens (Ajamda or Celery) heptobilary disorders.
37. Azadirachta indica (Nimba Neem).
38. Bacopa Monnieri (Brahmi or Thyme leaved Gratiola)
39. Berberis aristata (Daruharidra Tree Tumeric) Anti- inflammatory.
40. Boerhaavia diffusa ( Punarnava spreading hogweed) Anti inflammatory
41. Boswellia serata (Shallaki Boswellia) Commonly called Franckincense is used as a anti-inflammatory agent and improves immunity, it also has cancer reducing properties.
42. Butes monosperma (Palsa Butea Gum Tree) to trat heamorrage of the stomach and bladder.
43. Carpparis spinosa (Himsa the Caper Bush)
44. Caesalpina digyna Rottl (Udakiryaka or Teri pods)
45. Cassia fistula (Aragvadha or Indian Laburnum) used in blood poisoning, and in disorders of the live and biliousness.
46. Cassia occidentalis (Kasamarda or Negro Coffee)
47. Celastrus paniculatus (Jytishmati , Kanguni or Climing Staff tree) anti inflammatory.
48. Centella asiatica (Mdukapani Indian Pennywort, Gotu Kola)
49. Cicer arientinum (Chana or Bengal Gram, or Chickpea) contains polysaccharides
50. Chihorium intybus (Kasani, Chicory) hepatoprotective and is used in hepatic enlargement fever vomiting and abdominal pain.
51. Cinnamomum camphora (Karpoora Camphor) Used in circulation and respiration.
52. Cinnamomum cassia (Sthula tvak) Cassia Chinese Cinnamon used for stimulation of liver function.
53. cocus nufera (Narikela Coconut kernel) Used for its polysaccharides , its generaton of MDA a known agent in the fight against Aids and as an immunomodulator.
54. Crocus savitus (Kumkuma Saffron ) Used in liver treatment and stimulation of the nervous system.
55. Convolvulus microphyllus (Shankhapushpi) used as a psycho stimulant.
56. Cuminum cyminum ( Jeeraka, Cummin)
57. Curcuma longa (Haridra, Tumeric) Tumeric well known for its anti-inflammatory use and also more recently its effect on pancreatic cancer.
58. Cynodon dactylon (Durva, Dhub Grass)
59. Cyperus rotundus (Musta Nut Grass) a known anti-inflammatory.
60. Cyperus scariosus (Bhadramusta Umbellra's Edge )
61. Daucus carota (Shika-mula Carrot) The essential oils from leaves and seeds ae antifungal.
62. Didymocapus pedicellata (Shilapuspha)
63. Eclipta alba (Bhringaraja ) the plant possesses antiheptotoxic and anti inflammatory activities.
64. Elettaria cardamomum (Ela or Lesser Cardamom ) Anti-inflammatory.
65. Embelia ribes (Krimighna or False Black Pepper)
66. Emblica officinalis (Amalaki Or Indian Gooseberry)Contains CYTOKINE like substances identified as zeatin,z.riboside, z.nucleotide. (Part of Triphala)
67. Eucalyptus globules (Talipatra, Eucalyptus) pain relif
68. Gloriosa superba (Kalihari , Malabar Glory Lily)
69. Glycyrrhiza (Yashti -mahdu Liquorice) immuno stimulant properties.
70. Gossypium herbaceum (Karpassa Indian cotton ) nervine tonic.
71. Gymnema sylvestre ( Meshashringi Gymnema)
72. ipomoea digitata (vidari vidaarikaad or Milky Yam)
73. Holarrhena antidysenterica ( kutaja, Tellicherry Bark)
74. Nepeta hindostana (Billiotan, or Cal Mint)
75. Mucuna pruriens (Kapikachchhu or Cow Itch Plant ) L-dopa is neuro transmitter an effective drug for Parkinsons disease.
76. Myristica fragrans (Jatikosha, or Nutmeg) stimulates blood flow.
77. Mensua ferrea (Keshara , Or Ironwood Tree) Anti fungal anti Inflammatory and anti bacterial.
78. Mimosa pudica (Lajjalu or Touch me not) turgorines
79. Momordica charantia (Karavella or Bitter Gourd)
80. moringa pterygosperma (shigru, horse radish) anti-inflammatory, antispasmodic and antiviral
81. Mangifera indica( Amra , bark from the mango tree) Maniferine active component.
82. Hermidesmus indicus (Anantamul, Indian Sarsaparila) blood purifier.
83. Lens culinaris (Masura, Lentil) contain Flavonoids (Know for cancer inhibiting)
84. (Ksirakakoli Or White lily ) has soothing properties for hematemesis and is anti-inflammatory.
85. Small amount of oil of Cajuput from leaves of Cajuput Tree (antifungal)
86. Nerium odorum (Karavira or Sweet-Scented Oleander) anti-inflammatory.
87. Nigella sativa ( Cummin)
88. Olea europaea (Common Olive)
89. Peganum harmala (Harmala or Syrian Rue)
90. Phyllanthus amarus (Bhumyaamalaki) Used in the problems of the liver and kidney
91. Picrorhiza kurroa (Katuka)an immunomodulatory and immunostimulant having anti-inflammatory activity.
92. Piper longum (kana or Indian long pepper)
93. Piper nigrum (Black Pepper)
94. Evolvulus alsinoides (Vishnukrantha ) used as tonic and febrifuge
95. Gymnema sylvestre (meshashringi or gymnema) regenerative effect on pancreatic beta cells
96. Prunus amygdalus (Almond) nervine tonic
97. Pyrusmalus (Crab Apple)

The above three groups are immersed fully in organic acid/ fruit juices 3 as stated earlier. The group of fruit juices contain a combination of but are not limited to Grapefruit juice, grape juice, orange juice, lemon juice, water mellon plup, coconut juice/milk. (any seeds and acids from the skin would be included in the pot)

The whole would be pasteurised in the pot at between 55 to 65 degrees C , either by heating or by harnessing the power of the Sun.
During the process natural bacteria which can exist at low PH would convert some of the organic fruit acids to lactic acid, there would also be natural yeast in the air or on fruit bodies that would help ferment slightly the solution.

What is known is that the drink and food once cooked and especially the drink would be easily absorbed by the body and would contain a great many beneficial glyconutrients , that would help natural immunomodulation and boost the body functions, whilst flushing out toxins from the body. At the temperatures above few of the nutrients would be destroyed and others would be activated and extracted.
Alcohol produced would be extremely mild giving a gentle psychological boost.

The second Embodiment is perhaps controversial, however, consider that chemotherapy is effectively a poison and is not as shocking as it first seems or that a man having a serious motorcycle accident is more likely to survive when under the influence of alcohol than when he is sober.

The Amantia genus of mushrooms are used in this embodiment preferably the amatia muscaria or the fly agaric , although do not rule out using the more dangerous mushrooms if necessary when toxin levels can be tested, all would of course depend on the subject his metabolism , age, dosage etc.

It may be poison levels need to be increased to meet the disease concerned. Known as the Death Cap family of mushrooms they are highly toxic to all parts of the body especially the liver, the only defense is the milk thistle and penicillin, or a stomach pump in severe cases these mushrooms can cause death.

Amatia Muscaria therefore will be described for now as few have died from eating this mushroom and are added to the whole mix in the first embodiment.
There are reasons for the choice of poison it would have to cause necrosis in vivo, in order to activate the non-specific and older innate immune system namely the Dendrite cells by means of phagocytosis (see above) which would be a necessary activation step. The poison must also be flushed from the body as quickly as possible so as not to cause too much damage, and be protected against.

Amantia Muscaria , is added to the mix in embodiment 1. The liver and kidneys are protected by a large amount of milk thistle and nutrients that are in the same mix, penicillin could also be added after the mix is ready.

Milkthistle , also acts as an enema, it is also a natural liver regenerator i.e. allowing the poisons to be expelled quicker causing the liver to produce more bile which contains processed toxins and moves the bile out towards the small intestine for elimination. Freeing up the liver to process more incoming toxin that has accumulated , in the organs, issues and bloodstream (Max Gerson).

Only here the Amatia Muscaria is being extracted and taken at the same time and in the same solution as the extract of the milkthistle, not a half hour after.

Milkthistle a potential antioxidant in its own right is particularily remarkable for its beneficial effects on glutathione and increases levels by 35%, glutathione-s-transferase is an enzyme use by the liver to make the detox pathways run. Silymarin actually stimulates the production of new liver cells. Humans have 18 families of cyochrone p450 genesit is he mily CYP3 tha concerns this application most the grapefruit juice mentioned in the above embodient, contains naningin , and bergamottin which inhibit the cytochrome p450 Isoform CYP3A4 in the liver, it is via the inhibition of this enzyme that the grapefruit increases the affects of busiprone, caffine ,Nadine ,feldipin ,nifedipine etc increasing the effect of the production of bile by increasing the effect of coffee and milkthistle.

The grapefruit seed itself is antifungal. The mushroom was also picked for its beneficial effects the B-glucans and polysaccarides, please also see references above on immune system (WO2002/090559), Kidney function (CA2285900 ) and for its psychological nature, to stimulate the brain and nervous system to respond in a different way to allow the dendrite cells to mature and to allow regeneration because of the change /break of signals, or control.

However, here in a second embodiment a number of Psychoactive substances are used the Opium poppy is also added in the second embodiment, firstly because it stimulates the dendrite cells and secondly it produces histamines in vivo CA2461661 USE OF A HISTAMINE TO TREAT LIVER DISEASE. It is know also to have an active component that is beneficial to the pancreas.

Finally Cannabis is added this extract to add to the psychological effect and to restrict the growth of cancer and it also induces sleep in a slight overdose allowing sleep and therefore allowing regeneration to occur.

This second embodiment contains the whole of the first embodiment and adds the extra components as stated above.

It is necessary to understand that the first embodiment must be used first in patients who have had chemotherapy, or have weakened bodily systems as poison and toxins would already be present in the body. This before the second embodiment should even be considered for administration to Patients in the above category.

The combined psychological effect is necessary for regeneration, as it would stimulate the nervous and immune systems differently. Additionally the slightly alcoholic nature of the mix would cause the hormones in the kidneys to allow greater urination.

The mix of items in the groups within the first embodiment have been carefully chosen to counter the mal effects of the poison because of their anti inflammatory nature and should have the effect of alleviating nausea caused by the poison. The mix of items in the first embodiment are also anti fungal and beneficial to the liver and kidney functions.

Unfortunately those with reduced liver and kidney functions could not use the second embodiment unless correct dosages could be designed so the best way to make safe this remedy for mass use, may be in fact to generate or use the by products of a human volunteer that has taken the second embodiment together with the result of the mix from the first embodiment, i.e. therefore making the preferred third embodiment.

The preferred third embodiment would contain the by products (urine, faeces ) of the volunteer who has taken the second embodiment combined with the mix of the first embodiment, with the opium poppy and cannabis added to be extracted in the mix.

By increasing the bodies resistance due to poisoning the immune system would be forced to respond to an assumed attack, it would not respond to the poison directly only the affect of the poison therefore attacking the disease or cancer.
Stimulation of both the innate and adaptive immune systems occurs and the psychological changes allowing regeneration to occur more fully, by lessening the control on the immune system functions.The increased levels of Cd4+ cells would cause a remission also of Aids /Hiv. The increased production of MHC responders which are used in the therapy of autoimmune diseases, and the regeneration process would help in the autoimmune diseases.
It will be appreciated that variations in and modifications to the embodiments described and illustrated may be made within the scope of the invention.

## Claims

1. A method for producing, administrating a prophylactic or remedy for Cancer, HIV , AIDS and autoimmune diseases the method comprising pasteurising mushrooms, vegetables , herbs, seeds, roots, grasses , flowers, tree bark , leaves and ayurvedic medicines in natural organic and fruit juices to extract nutrients to produce a slightly alcoholic drink for consumption.

2. A system for producing, administrating a prophylatic or remedy for Cancer, Aids, HIV and autoimmune diseases comprising a means to heat mushrooms, vegetables, herbs, seeds , roots, grasses , flowers, tree bark, leaves and ayurvedic medicines immersed in natural organic and fruit juices at temperatures effective to extract nutrients by pasteurisation;

3. A pharmaceutical composition comprising a therapeutically effective amount of poison its antidote and at least one psychologically active agent coupled with glyconutrients and antioxidants or derivative, homologue or fragment thereof for the treatment of Cancer, HIV, Aids, and autoimmune diseases.

4. A method for producing, a non-toxic food/drink for boosting immune system, and detox comprising pasteurising mushrooms, vegetables, herbs, seeds , roots, grasses , flowers, tree bark, leaves and ayurvedic medicines in natural organic and fruit juices to extract nutrients to produce a slightly alcoholic drink for consumption.

5. A system for producing, a non toxic mushroom food /drink for boosting the immune system and detox comprising a means to heat mushrooms, vegetables, herbs, seeds, roots, grasses, flowers, tree bark, leaves and ayurvedic medicines immersed in natural organic and fruit juices at temperatures effective to extract nutrients by pasteurisation for consumption;

6. A method of extraction of medicines from mushrooms, vegetables , herbs, seeds , roots, grasses, flowers, tree bark, leaves and ayurvedic medicines plants by organic acids and natural fermentation at temperatures effective to pasteurise.

7. A system to extract for the use in medicine making the active components from mushrooms, vegetables, herbs, seeds, roots, grasses , flowers, tree bark , leaves and ayurvedic medicines plants by using organic acids and natural fermentation at temperatures effective to pasteurise.

8. A method according to claim 1 for producing, administrating a prophylactic or remedy for Cancer, HIV, AIDS and autoimmune diseases the method comprising extraction of the amantia family of mushrooms preferably amantia muscaria, milkthistle, opium poppy, cannabis together vegetables , herbs, seeds , roots, grasses, flowers, tree bark, leaves and ayurvedic medicines in grapefruit juice and other natural organic and fruit juices to extract nutrients to produce a slightly alcoholic drink for consumption.

9. A system according to claim 2 for producing, administrating a prophylatic or remedy for Cancer, Aids, HIV and autoimmune diseases comprising a means to heat the amantia family of mushrooms preferably the amantia muscaria mushrooms together with other non-toxic mushrooms vegetables , herbs, seeds , roots, grasses, flowers, tree bark, leaves and ayurvedic medicines by immersing in natural organic and fruit juices at temperatures effective to extract nutrients by pasteurisation;

10. A method according to claim 8 for producing, administrating a prophylatic or remedy for Cancer, Aids, HIV and autoimmune diseases comprising the byproducts of the amantia mushrooms preferably the amantia muscaria mushrooms from the human body together with other non-toxic mushrooms vegetables, herbs, seeds , roots , grasses , flowers, tree bark , leaves and ayurvedic medicines extracted by immersing in natural organic and fruit juices at temperatures effective to extract nutrients by pasteurisation;
